# EUROPEAN PATENT APPLICATION

(11) **EP 1 433 845 A1**
(43) Date of publication of application: **30.06.2004**
(21) Application number: 02028812.2
(22) Date of filing: 23.12.2002
(51) Int. Cl.: C12N 9/52, C12N 15/57, C12N 15/11, C12N 1/21, C07K 16/40, A61K 38/48

(54) **Clostridium tetani collagenase and uses thereof**

(71) Applicant: Georg-August-Universität, 37077 Göttingen (DE)
(72) Inventor: Brüggemann, Holger, 37073 Göttingen (DE); Bäumer, Sebastian, 37073 Göttingen (DE); Gottschalk, Gerhard, 37176 Nörten-Hardenberg (DE); Hartsch, Thomas, 07745 Jena (DE)
(74) Representative: Grund, Martin, Dr.

(57) **Abstract**

This invention relates to a collagenase derived from *Clostridium tetani,* its amino acid sequence, analogues and variants thereof. Furthermore, the invention is directed to isolated nucleic acid sequences encoding said collagenase, its analogues and variants, their expression and uses of the thus expressed proteins for cell culture and therapeutic applications.

SEQUENCE LISTING

<110>  Georg-August-Universität

<120>  Clostridium tetani collagenase and uses thereof

<130>  337-2

<140>  ..................
<141>  2002-12-23

<160>  2

<170>  PatentIn version 3.1

<210>  1
<211>  991
<212>  PRT
<213>  Clostridium tetani

<400>  1

Met Lys Lys Lys Phe Ile Lys Met Leu Cys Ser Ile Ala Ile Gly Cys
1               5                   10                  15

Met Ile Ser Thr Ser Tyr Ser Ile Lys Val Ser Ala Phe Ser Asn Gly
            20                  25                  30

Asn Thr Lys Thr Asn Pro Asn Gly Glu Phe Lys Ser Leu Ser Leu Asn
        35                  40                  45

Ser Thr Asn Pro Tyr Lys Thr Lys Tyr Ser Phe Asn Asp Leu Asn Lys
    50                  55                  60

Leu Ser Asn Lys Glu Ile Leu Asp Leu Thr Ser Lys Ile Lys Trp Ser
65                  70                  75                  80

Asp Ile Ser Asp Leu Phe Gln Tyr Asn Lys Asp Ser Tyr Thr Phe Tyr
                85                  90                  95

Ser Asn Lys Glu Arg Val Gln Ala Leu Ile Asp Gly Leu Tyr Glu Lys
            100                 105                 110

Gly Cys Asn Tyr Thr Ser Thr Asp Asp Lys Gly Ile Asp Thr Leu Val
        115                 120                 125

Glu Ile Leu Arg Ser Gly Phe Tyr Leu Gly Tyr Tyr Asn Asp Ser Leu
    130                 135                 140

Lys Tyr Leu Asn Asp Lys Ser Phe Lys Asp Lys Cys Ile Pro Ala Met
145                 150                 155                 160

Ile Ala Ile Glu Asn Asn Lys Asn Phe Lys Leu Gly Glu Asn Gly Gln
                165                 170                 175

Asp Thr Val Val His Ala Leu Gly Lys Leu Ile Gly Asn Thr Ser Cys
            180                 185                 190

Asn Asp Glu Val Val Asn Lys Thr Ile Pro Ile Leu Glu Gln Tyr Tyr
        195                 200                 205

Asn Glu Ile Asp Lys Tyr Ser Lys Asp Arg Leu Lys Ser Asn Ala Val
    210                 215                 220

Tyr Asn Phe Met Lys Glu Ile Asn Tyr Asp Ile Ser Gln Tyr Glu Tyr
225                 230                 235                 240

Ala His Asn Ile Arg Asp Tyr Lys Asn Thr Pro Trp Ser Gly Lys Ile
                245                 250                 255

Asp Ser Phe Ile Asp Thr Ile Ser Lys Phe Ala Ser Ile Ser Asn Val
            260                 265                 270

Thr Lys Asp Asn Gly Trp Ile Ile Asn Asn Ser Ile Tyr Tyr Thr Ala
        275                 280                 285

Lys Leu Ser Lys Tyr His Ser Asn Pro Ser Ile Pro His Ser Val Ile
    290                 295                 300

Asp Asn Cys Ile Glu Ile Phe Pro Asp Tyr Ser Glu Gln Tyr Phe Thr
305                 310                 315                 320

Ala Ile Glu Ala Ile Lys Glu Asp Phe Asn Ser Arg Asp Ser Lys Gly
                325                 330                 335

Asn Val Ile Asp Ile Asn Lys Leu Ile Glu Glu Gly Lys Lys His Tyr
            340                 345                 350

Leu Pro Lys Thr Tyr Thr Phe Asp Asn Gly Lys Ile Ile Ile Lys Ala
        355                 360                 365

Gly Asp Lys Val Glu Glu Ser Lys Ile Gln Lys Leu Tyr Trp Ala Ser
    370                 375                 380

Lys Glu Val Lys Ser Gln Phe His Arg Ile Ile Gly Asn Asp Lys Pro
385                 390                 395                 400

Leu Glu Val Gly Asn Ala Asp Asp Ile Leu Thr Ile Val Ile Tyr Asn
                405                 410                 415

Asn Pro Glu Glu Tyr Lys Leu Asn Lys Thr Leu Tyr Gly Tyr Ser Val
            420                 425                 430

Asp Asn Gly Gly Ile Tyr Ile Glu Gly Ile Gly Thr Phe Phe Thr Tyr
        435                 440                 445

Glu Arg Thr Pro Gln Glu Ser Ile Tyr Ser Leu Glu Glu Leu Phe Arg
    450                 455                 460

His Glu Phe Thr His Tyr Leu Gln Gly Arg Tyr Leu Ile Pro Gly Leu
465                 470                 475                 480

Phe Asn Lys Gly Asp Phe Tyr Lys Gly Asn Asn Gly Arg Ile Thr Trp
                485                 490                 495

Phe Glu Glu Gly Ser Ala Glu Phe Phe Ala Gly Ser Thr Arg Thr Ser
            500                 505                 510

Val Leu Pro Arg Lys Ser Met Val Gly Gly Leu Ser Lys Asn Pro Lys
        515                 520                 525

Glu Arg Phe Asn Ala Asp Lys Leu Leu His Ser Lys Tyr Ser Asp Gly
    530                 535                 540

Trp Asp Phe Tyr Lys Tyr Gly Tyr Ala Phe Ser Asp Tyr Met Tyr Asn
545                 550                 555                 560

Asn Asn Lys Lys Leu Phe Ser Asp Leu Val Ser Thr Met Lys Asn Asn
                565                 570                 575

Asp Val Lys Gly Tyr Glu Ala Leu Ile Glu Glu Ser Ser Lys Asp Ser
            580                 585                 590

Lys Ile Asn Lys Asp Tyr Glu Tyr His Met Glu Asn Leu Val Asn Asn
        595                 600                 605

Tyr Asp Asn Tyr Thr Ile Pro Leu Val Ser Asp Asp Tyr Met Lys Gln
    610                 615                 620

Tyr Asp Asn Lys Ser Leu His Glu Ile Lys Ser Asp Ile Glu Lys Ala
625                 630                 635                 640

Met Asp Val Lys Asn Ser Gln Ile Thr Lys Glu Ser Ser Gln Tyr Phe
                645                 650                 655

Asp Thr Tyr Asn Leu Lys Ala Thr Tyr Thr Leu Ser Ser Asn Lys Gly
            660                 665                 670

Glu Ile Ser Asn Trp Asn Tyr Met Asn Asn Lys Ile Asn Glu Ala Leu
        675                 680                 685

Asn Lys Leu Asp Asn Leu Ser Trp Gly Gly Tyr Lys Thr Val Thr Ala
    690                 695                 700

Tyr Phe Ser Asn Pro Arg Leu Asn Ser Asn Asn Glu Val Val Tyr Asp
705                 710                 715                 720

Ile Val Phe His Gly Leu Leu Ser His Asn Lys Asn Ser Asn Glu Lys
                725                 730                 735

Val Glu Val Lys Glu Glu Pro Glu Ile Lys Asp Lys Asp Ser Phe Glu
            740                 745                 750

Asn Val Ile Tyr Glu Lys Glu Asn Asn Asp Ser Phe Asp Lys Ala Asn
        755                 760                 765

Lys Ile His Lys Asn Gln Ile Val Met Ala Thr Leu Asp Thr Glu Asp
    770                 775                 780

Tyr Arg Asp Thr Phe Tyr Phe Asp Ala Leu Thr Ser Gly Ser Ile Asp
785                 790                 795                 800

Ile Thr Ile Glu Asn Ile His Gly Asn Ser Asp Ala Phe Asn Trp Leu
                805                 810                 815

Val Tyr Asn Asp Glu Asp Leu Asn Asn Tyr Ile Ala Tyr Pro Thr Lys
            820                 825                 830

Lys Glu Asp Asn Lys Leu Met Gly Ser Phe Lys Val His Lys Pro Gly
        835                 840                 845

Arg Tyr Tyr Ile Leu Val Tyr Lys Thr Ser Leu Asn Lys Val Asn Tyr
    850                 855                 860

Lys Leu Asn Ile Ser Asp Ala Thr Asn Met Ala Pro Val Ile Lys Lys
865                 870                 875                 880

Ile His Glu Lys Glu Asn Asn Asp Ser Phe Glu Thr Ala Asn Lys Ile
                885                 890                 895

Thr Leu Asp Thr Leu Val Leu Gly Asn Leu Asp Tyr Lys Asp Val Ser
            900                 905                 910

Asp Ile Tyr Ser Phe Asp Ile Glu Asn Thr Lys Asp Leu Asn Ile Lys
        915                 920                 925

Leu Thr Asn Leu Asn Asn Leu Gly Ile Ala Trp Asn Leu Tyr Lys Glu
    930                 935                 940

Ser Asp Leu Asn Asn Tyr Ile Ala Tyr Gly Ala Lys Ser Asp Asn Ala
945                 950                 955                 960

Ile Val Gly Lys Cys Asn Leu Ser Pro Gly Lys Tyr Tyr Leu Tyr Val
                965                 970                 975

Tyr Lys Tyr Ser Gly Asp Lys Gly Asn Tyr Ser Val Ile Ile Asn
            980                 985                 990

<210>  2
<211>  3079
<212>  DNA
<213>  Clostridium tetani

<400>  2
tataatgtat aatgtattca tttaatattt attataaatt taattaaaat ttaaattaaa      60

tattaaagta aatataatat ttataatgtg aggtattaat atgaaaaaaa aatttattaa     120

aatgctatgt tccatagcta taggttgtat gataagtaca agctattcaa ttaaagtatc     180

tgctttttct aatggcaata ctaaaactaa cccaaatggt gaatttaaat ccttaagttt     240

aaattctaca aacccctaca aaacaaagta ttcttttaat gatttaaaca aactaagtaa     300

taaggaaatt ttagatttaa cttcaaaaat aaaatggagt gatatttctg atctttttca     360

gtataacaag gatagttata ccttttattc aaacaaagaa cgtgtacaag ctttaataga     420

tggattatat gaaaaaggtt gtaattatac tagcactgat gacaaaggaa ttgatacatt     480

agttgaaatt ttaagatctg gattttattt aggatattat aatgattctt taaaatactt     540

aaatgataaa tcttttaagg ataaatgtat tccagcaatg attgctatag aaaataataa     600

aaatttcaaa ctaggtgaaa atggtcaaga tacagtagta catgctttag gtaaacttat     660

aggaaatact tcttgcaatg atgaagtagt taataaaaca attcctattt tagaacaata     720

ttataatgaa atagataagt attcaaagga tagactaaaa tccaatgctg tatataattt     780

tatgaaagaa ataaattatg atatttcaca atatgaatat gctcataata ttagagatta     840

taaaaatact ccttggtctg gtaaaataga ttcctttata gatacaattt caaaatttgc 900
aagtatatct aatgttacaa aggataatgg ctggattata aataatagta tttattatac 960
tgcaaaactt tcaaaatacc acagtaatcc atctattcct cattcagtaa tagataattg 1020
cattgaaatt tttcctgatt atagcgaaca atattttact gcaatagagg caataaagga 1080
agattttaat tcaagggact caaagggcaa tgttatagac attaataagt taatagaaga 1140
aggtaaaaaa cattatctac ctaaaactta tacctttgac aatggaaaaa tcattataaa 1200
ggctggagat aaagtagaag aatcaaaaat tcaaaaactt tactgggcat ctaaagaagt 1260
aaaatctcaa tttcatagga taataggtaa tgataaaccc ctagaggtag gcaatgctga 1320
tgatatatta actatagtaa tatacaacaa tccagaagaa tataaactaa acaaaacttt 1380
atatggatat agtgtagata atggtggaat atatattgaa ggaattggta ctttctttac 1440
ctatgaaaga actccacagg aaagcatcta tagcctagaa gaactttttc gtcatgaatt 1500
cactcattat ttacaaggac gttacttaat accaggtcta tttaataaag gtgattttta 1560
caaaggaaat aatggaagaa ttacatggtt tgaagaaggc tctgcagaat tttttgcagg 1620
ttcaactaga acttctgtat taccaagaaa atcaatggta ggtggacttt ctaaaaatcc 1680
taaagaaaga tttaatgcag ataaattatt acattcaaaa tatagtgatg gatgggattt 1740
ttataaatat ggatatgctt tctcagatta tatgtataat aacaacaaga aattatttag 1800
tgatttagta tctaccatga aaaataatga tgttaaaggt tatgaagctt taatagaaga 1860
atcaagtaaa gattctaaaa ttaataaaga ctatgaatat catatggaaa acttagttaa 1920
taattatgat aactacacaa taccattagt ttctgatgat tatatgaaac aatatgataa 1980
taaatcttta catgaaataa aatcagatat tgaaaaagct atggatgtaa aaaattctca 2040
aataactaaa gaaagttccc aatactttga tacctataat ttaaaagcaa cttatacatt 2100
aagttcaaat aaaggggaaa tttctaattg gaattatatg aataataaaa ttaacgaagc 2160
tttaaataaa ctagataatt taagctgggg aggatataaa acagtaactg cttatttctc 2220
aaatcctaga ttaaattcaa ataatgaagt agtatatgat atagttttcc atggattatt 2280
atctcataat aaaaattcaa atgaaaaggt agaagttaaa gaggagccag aaattaaaga 2340
taaggattcc tttgaaaatg taatatatga aaaagaaaat aacgattctt ttgataaagc 2400
taataaaatt cataaaaacc aaatagtaat ggctacttta gatactgagg actacaggga 2460
caccttttac tttgatgctc taacctctgg aagtatagat ataactatag aaaacattca 2520
tggtaattct gatgcattta attggcttgt ttacaatgat gaggatttaa ataattatat 2580
tgcataccct acaaaaaaag aagataataa acttatgggc agttttaaag tacataaacc 2640
tggtagatat tatatattag tttataaaac ctctttaaat aaagtaaatt ataagttaaa 2700
tataagtgat gctactaata tggctccagt aataaaaaaa attcatgaaa aagaaaataa 2760
tgattctttc gaaactgcaa ataaaattac acttgatacc cttgtattag gtaacctaga 2820
ttataaggat gttagtgata tttattcatt tgatatagaa aatactaagg atttaaatat 2880
aaagctaact aatttaaata atttagggat agcctggaac ctttataaag aatcagattt 2940
aaataattat attgcctatg gagcaaaatc agataatgct atagtaggta aatgtaattt 3000
aagtcctggg aaatattatt tatatgtgta taaatactct ggtgataagg gtaattattc 3060
agtaataata aattaatgc 3079

## Description

This invention relates to a collagenase derived from *Clostridium tetani,* its amino acid sequence, analogues and variants thereof. Furthermore, the invention is directed to isolated nucleic acid sequences encoding said collagenase, its analogues and variants, their expression and uses of the thus expressed proteins.

### Background of the invention

Tetanus disease is one of the most dramatic and globally prevalent diseases of humans and vertebrate animals, reported for over 24 centuries. The manifestation of the disease, spastic paralysis, is caused by the second most poisonous substance known, the tetanus toxin, with a human lethal dose of about 1 ng/kg. Fortunately, this disease is successfully controlled through immunization with tetanus toxoid; nevertheless, according to the World Health Organization (WHO) an estimated 400,000 cases still occur each year, mainly of neonatal tetanus.

The causative agent of tetanus disease is *Clostridium tetani,* an anaerobic spore-forming bacterium, whose natural habitat is soil, dust and intestinal tracts of various animals.

Neurotoxigenic clostridia have attracted considerable attention during the past decades. The mode of action of the strongest toxins known to mankind, the tetanus toxin produced by *Clostridium tetani* and the homologous botulinum toxins A-G produced by various *C. botulinum* strains are now well understood (Tizzoni, G. & Cattani, G. (1890) *Zentralbl. Bakt.* 8, 69-73; Montecucco, C. & Schiavo, G. (1993) *Trends Biochem Sci* 18, 324-327; Swaminathan, S. & Eswaramoorthy, S. (2000) *Nature Struct. Biol.* 7, 693-699; Arnon, S. S. (1997) in *The clostridia: molecular* *biology and pathogenesis,* eds. Rood, J. I., McClane, B. A., Songer, J. G. & Titball, R. W. (Academic press, San Diego), pp. 95-115). The tetanus toxin (TeTx) blocks the release of neurotransmitters from presynaptic membranes of inhibitory neurons of the spinal cord and the brainstem of mammals; it catalyses the proteolytic cleavage of the synaptic vesicle protein synaptobrevin (Schiavo, G. et al., (1992) *Nature* 359, 832-835). This leads to continuous muscle contractions, which are primarily observed in jaw and neck muscles (lockjaw). Since the introduction of a potent vaccine during World War II, cases of tetanus disease have only been sporadic in industrial countries. However, the disease, and in particular neonatal tetanus (NT), is still an important cause of death due to insufficient immunization (Arnon, S. S. (1997) in *The clostridia: molecular biology and pathogenesis,* eds. Rood, J. I., McClane, B. A., Songer, J. G. & Titball, R. W. (Academic press, San Diego), pp. 95-115; Gasse, F. (1995) *Int J Gynaecol Obstet* 50, S67-S72). NT is the second leading cause of death from vaccine-preventable diseases among children worldwide. It is considered endemic to 90 developing countries, and an estimated 248,000 deaths occurred in 1997 (WHO; http://www.who.int/vaccines-diseases/diseases/NeonatalTetanus.shtml).

So far, only very little genetic information on *C. tetani* is available. Basically, said information is mainly restricted to the nucleotide sequences of TeTx and of its direct transcriptional activator TetR, both of which are encoded on a plasmid, designated pCL1 in the strain Massachusetts (Eisel, U., et al., (1986) *EMBO J* 5, 2495-2502; Fairweather, N. F. & Lyness, V. A. (1986) *Nucleic Acids Res.* 14, 7809-7812; Marvaud, J.-C., et al., (1998) *Infect. Immun.* 66, 5698-5702; Finn, C. W., et al., (1984) *Science* 224, 881-884).

Accordingly, in order to provide a better understanding of the lifestyle switch from a harmless soil bacterium to a potentially devastating neurosystem-damaging organism after entering and infecting a mammalian host, it is an object of the present invention to identify and analyse all genes in the genome of *C. tetani.*

This is accomplished by providing, for the first time, the complete genome sequence of toxigenic *C. tetani* E88, a variant of strain Massachusetts. The genome consists of a 2,799,250 base pairs (bp) chromosome encoding 2,372 open reading frames (ORFs). The tetanus toxin is encoded on a 74,082 bp plasmid, pE88, containing 61 ORFs.

Surprisingly, said *C. tetani* plasmid also encodes a collagenase, which plays an important role in *C. tetani* pathogenesis.

So far, *Clostridium histolyticum* collagenase is the best studied bacterial collagenase (Mookhtiar, K. A. & Van Wart, H. E. (1992) *Matrix Suppl.* 1, 116-126) and is widely used as a tissue dispersing enzyme (Seglen, P. O. (1976) *Methods Cell Biol.* 13, 29-83; Worthington, C. C. (1988) *Worthington Enzyme Manual: Collagenase,* Worthington Biochemical Co., Freehold, NJ). Other types of collagenases, which differ in substrate specificity and molecular structure, have also been identified. Bacterial collagenases differ from vertebrate collagenases in that they exhibit broader substrate specificity (Peterkofsky, B. (1982) *Methods Enzymol.* 82, 453-471; Birkedal-Hansen, H. (1987) *Methods Enzymol.* 144, 140-171).

However, a *C. tetani* collagenase was yet unknown. Accordingly, a further object of the invention consist in the provision of the amino acid and nucleic acid sequence of said *C. tetani* collagenase.

Furthermore, the present invention also contributes to unravel the regulatory network governing tetanus toxin formation, which is still poorly understood (Marvaud, J-C., et al., (2000) *Biol. Cell.* **92,** 455-457.).

Additional virulence-related factors could be identified, such as an array of surface-layer and adhesion proteins (35 ORFs), some of them unique to *C. tetani.* Comparative genomics with the genomes of *C. perfringens,* the causative agent of gas gangrene, and *C. acetobutylicum,* a non-pathogenic solvent producer, revealed a remarkable capacity of *C. tetani:* the organism can rely on an extensive sodium ion bioenergetics.

Further candidate genes involved in the establishment and maintenance of a pathogenic lifestyle of *C. tetani* are presented.

### Short description of the drawings

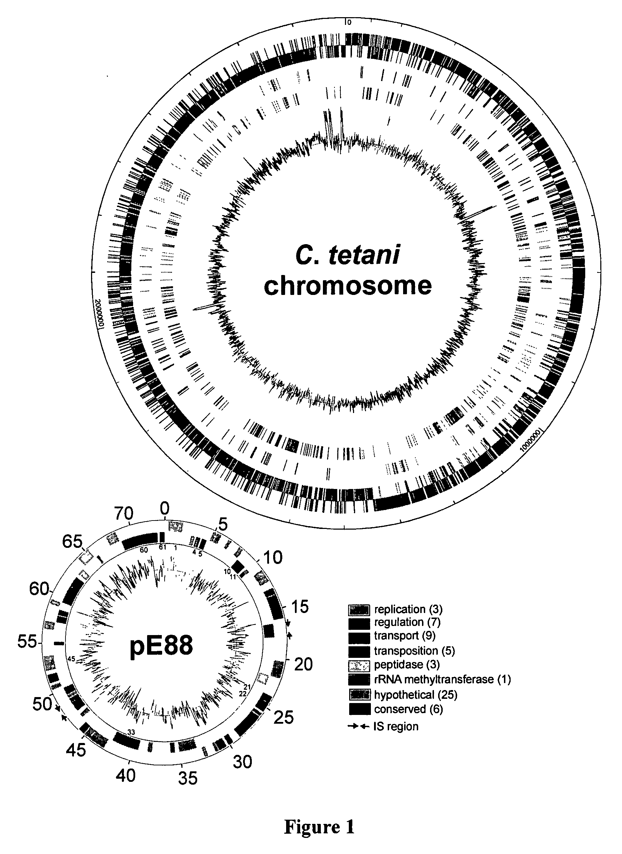

**Figure 1: Circular maps of the chromosome and the plasmid pE88 of *C. tetani.*** The plasmid map shows ORFs color-coded according to their assigned functions. Numbers at the inner ring refer to genes mentioned in the text. Most inner rings of both maps show the G+C content variation (higher values outward).

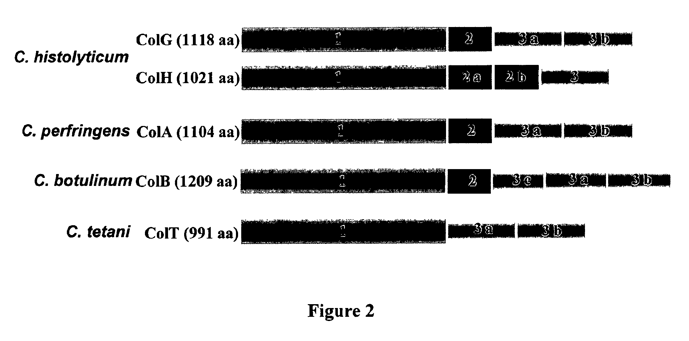

**Figure 2: Comparison of clostridial collagenases.** Segment comparison of the collagenases of *C. perfringens* (Matsushita, O., et al., (1994) *J. Bacteriol.* 176, 149-156), *C. histolyticum* (Matsushita, O., et al., (1999) *J. Bacteriol.* 181, 923-933), *C. botulinum* (Sanger Centre, *C. botulinum* sequencing project; online access: ftp://ftp.sanger.ac.uk/pub/pathogens/cb/), and *C. tetani.* Bacterial collagenases consist of three different segments (Matsushita, O., et al., (1999) *J. Bacteriol.* 181, 923-933): Segment 1 represents the catalytic domain containing the consensus motif for zinc proteases, HE*XX*H. Segment 2 contains the so-called PKD domain (PF00801) of unknown function. The name refers to the polycystic kidney disease protein PKD 1, in which the domain was detected in multiple copies. Segment 3 is thought to be the collagen-binding domain (CBD).

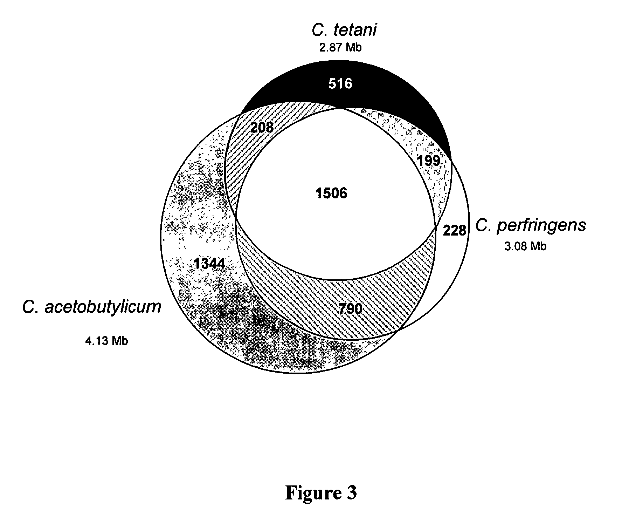

**Figure 3: Occurrence of homologous ORFs in *C. tetani, C. perfringens* and *C. acetobutylicum.*** Clostridial "backbone" ORFs, 1506; ORFs in *C. tetani* with homologues in *C. perfringens* but not in *C. acetobutylicum,;* 199, *C. tetani* ORFs present in *C. acetobutylicum* but absent in *C. perfringens,* 1344; *C. tetani* ORFs not present in *C. perfringens* and *C. acetobutylicum,* 516. The size of the circles is proportional to the number of ORFs in each organism.

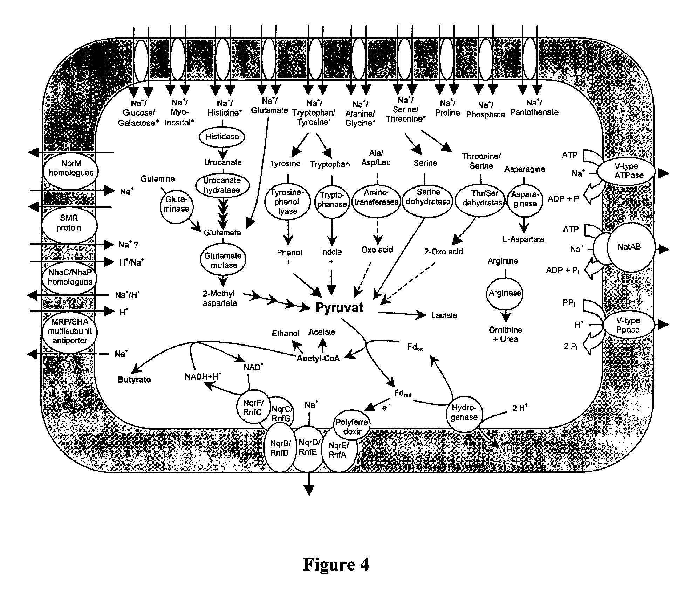

**Figure 4: Role of Na+ in amino acid fermentations by *C. tetani.*** Information on the NQR/RNF electron transport system is given in the supporting information. The substrate specificity of many Na+-dependent symporters (tagged with *) remains obscure (Paulsen, I. T., et al., 2000) *J. Mol. Biol.* 301, 75-100). Abbreviations: V-type ATPase, vacuolar-type ATPase; V-type Ppase, vacuolar-type pyrophosphatase; NatAB, an ABC (ATPbinding cassette) transporter (Häse, C. C., et al., (2001) *Microbiol. Mol. Biol. Rev.* 65, 353-370); Fd, Ferredoxin; NorM, a Na+-translocating multidrug transporter characterized in *Vibrio parahaemolyticus* (Morita, Y., et al., (2000) *J. Bacteriol.* 182, 6694-6697); SMR, Small multidrug resistance family; NhaC and NhaP, one-subunit secondary H+/Na+ antiporters; MRP/SHA, this seven-subunit H+/Na+ antiporter with similarities to hydrophobic components of the respiratory Complex I has been characterized in *Bacillus subtilis* and *Staphylococcus aureus* (Ito, M., Guffanti, A. A. & Krulwich, T. A. (2001) *FEBS Lett.* 496, 117-120).

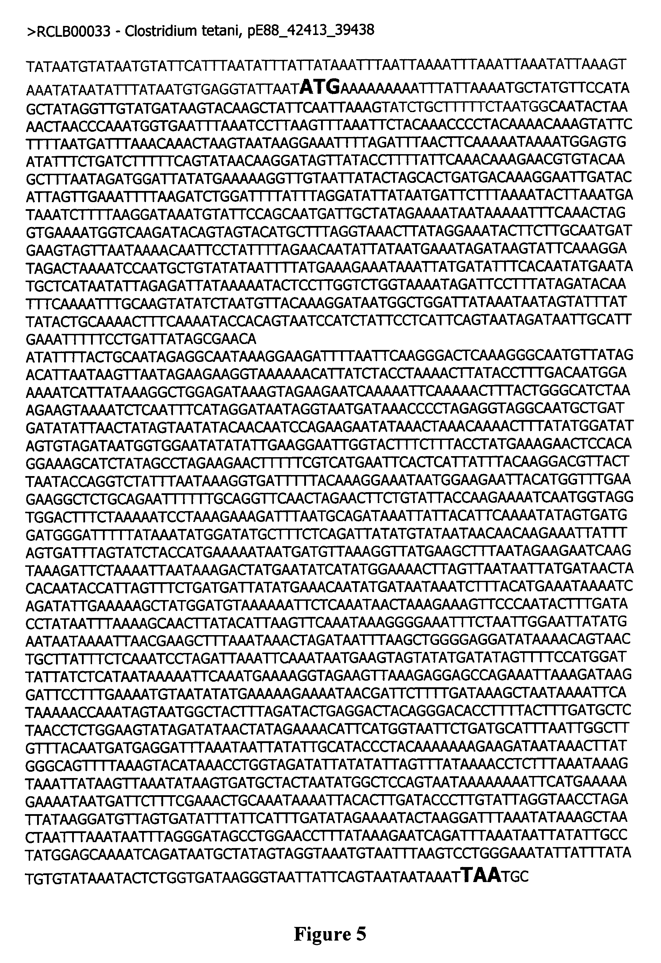

**Figure 5: Nucleic acid sequence of *C. tetani* collagenase.** Fig. 5 shows the nucleic acid sequence of *C. tetani* collagenase, located at bp 42,413 to 39,438 on plasmid pE88.

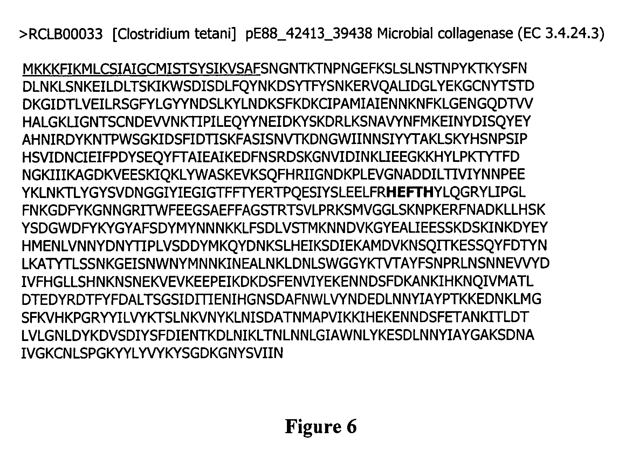

**Figure 6: Amino acid sequence of *C. tetani* collagenase.** Fig. 6 shows the deduced amino acid sequence of the *C. tetani* collagenase according to the invention. The *C. tetani* collagenase is characterized by an amino acid sequence of 991 amino acids with a calculated molecular weight of 114,375 kDa. *C. tetani* collagenase is translated as a propeptide and is cleaved upon extracellular transport by a signal peptidase in order to yield the mature protein. The cleavage site is located between amino acids A and F of the signal peptide, which is underlined. The HexxH consensus motif of all zinc proteases is shown in bold (amino acids 465-469).

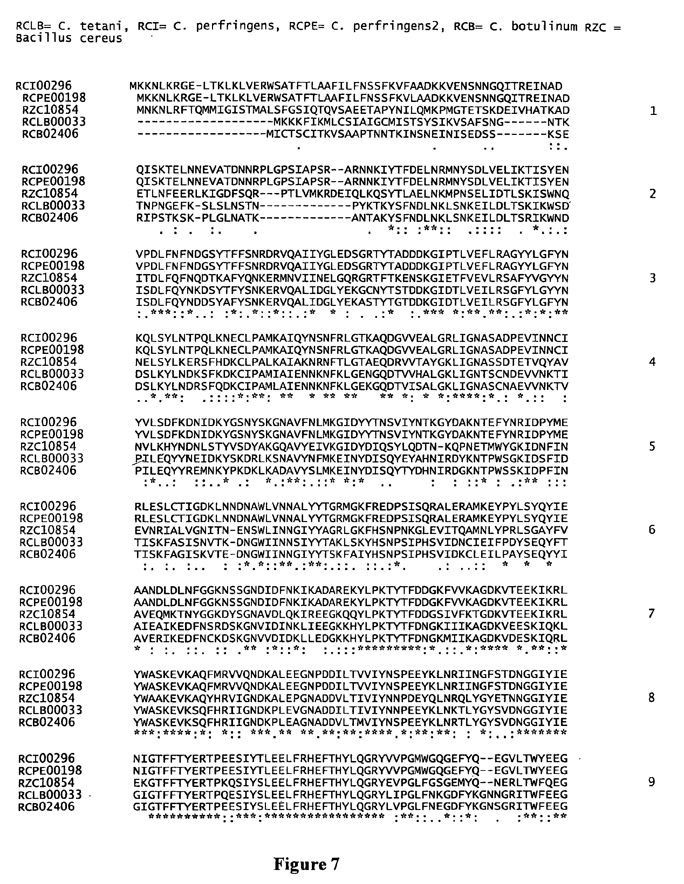

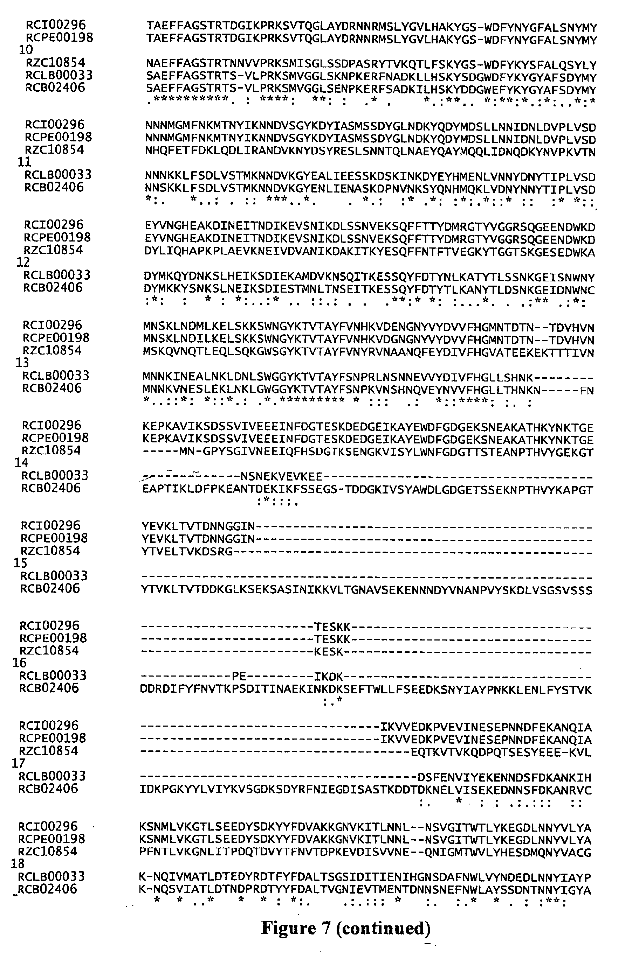

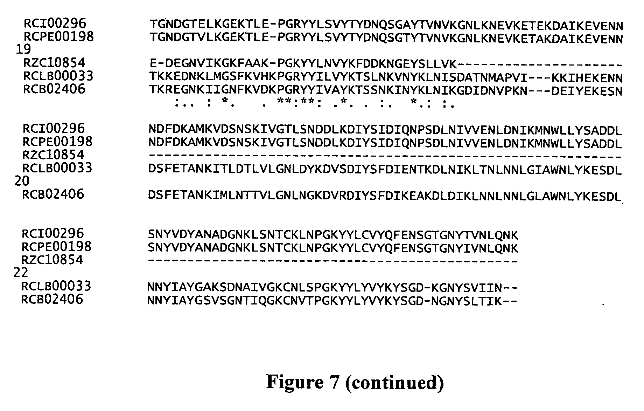

**Figure 7: Alignment of different collagenases.** Fig. 7 shows an amino acid alignment of collagenases from *C. tetani* (RCLB), *C. perfringens* (RCI), *C. perfringens2* (RCPE), *C. botulinum* (RCB), *Bacillus cereus* (RZC).

### Disclosure of the invention

The invention is directed to a collagenase derived from a bacterium of the species *Clostridium tetani.*

The term "collagenase" denotes proteolytic enzymes which are capable of degrading native collagen. Collagenases are produced by microorganisms and may be released in a proenzyme form into tissues and require activation by other proteases before they will degrade fibrillar matrix.

Referring to clostridial collagenases, they were reported to consist of three different segments (Matsushita, O., et al., (1999) *J. Bacteriol.* 181, 923-933; see also Fig. 2): While segment 1 represents the catalytic domain containing the consensus motif for zinc proteases, HE*XX*H, segment 2 contains the so-called PKD domain (PF00801) of unknown function. Said denomination refers to the polycystic kidney disease protein PKD1 (Bycroft M. et al., *EMBO J.* 18(2), pp. 297-305; *Tsiokas L, et al., Proc Natl Acad Sci U S A 1997 Jun 24;94(13)),* in which the domain was detected in multiple copies. Segment 3 is thought to be the collagen-binding domain (CBD).

Surprisingly, the collagenase of the present invention does not contain the PKD domain. Accordingly, a preferred embodiment of the invention is directed to a collagenase derived from *C. tetani* comprising a catalytic domain and a collagen-binding domain. In a particularly preferred embodiment, the invention is directed to a collagenase derived from *C. tetani* comprising a catalytic domain and a collagen-binding domain, but lacking the PKD domain.

More specifically, the collagenase according to the present invention is characterized by the amino acid sequence of SEQ ID NO: 1. Additionally, the invention is also directed to an isolated nucleic acid sequence represented by SEQ ID NO.2, which encodes a collagenase characterized by the amino acid sequence of SEQ ID NO.1.

Since the present invention advantageously provides for the first time an isolated nucleic acid sequence encoding a *C. tetani* collagenase, the person skilled in the art is provided with the ability to manipulate the thus encoded collagenase. Consequently, the invention also provides collagenase variants, which are characterized by amino acid substitutions, deletions or additions. In a preferred embodiment, said variants retain the biological activity of the naturally occurring collagenase.

In another embodiment, the invention is also directed to analogues of the collagenase as represented by amino acid sequence SEQ ID NO: 1.

Furthermore, the invention permits production of *C. tetani* collagenase via the use of recombinant expression vectors comprising an isolated nucleic acid sequence encoding a collagenase according to the present invention. The invention also provides host cells transformed with the recombinant expression vector, wherein said host cells may be of bacterial, yeast or insect origin. Upon cultivating the transformed host cells and expression of the collagenase, the thus produced protein may be recovered.

The invention is also directed to an isolated antibody that binds specifically to the collagenase according to the present invention.

Finally, the invention is also directed to the use of the collagenase. In one embodiment, the invention is directed to the use of *C. tetani* collagenase for the isolation of viable cells from tissue. Another embodiment relates to *C. tetani* collagenase for use in the treatment of diseases associated with excessive or abnormal collagen deposition. Further preferred embodiments are directed to the *C. tetani* collagenase for use as a treatment of injuries such as burn, wounds, ulcer, scab, white hard scab of collagen base, cheloid, necrosis, in particular necrosis by decubitus or ulcer.

### Detailed description of the invention

### C. tetani collagenase

The invention is directed to a collagenase derived from *C. tetani.* Said collagenase was cloned from the 74,082 bp tetanus toxin-encoding plasmid pE88 from toxigenic *C. tetani* E88, a variant of strain Massachusetts.

Regarding the 74 kb *C. tetani* plasmid pE88, it was known to harbor the genes for the tetanus toxin (*tet*X, CTP60) and its direct transcriptional regulator TetR (Eisel, U., et al., (1986) *EMBO J.* 5, 2495-2502; Fairweather, N. F. & Lyness, V. A. (1986) *Nucleic Acids Res.* 14, 7809-7812; Marvaud, J.-C., et al., (1998) *Infect. Immun.* 66, 5698-5702; Finn, C. W., et al., (1984) *Science* 224, 881-884). The present invention now identifies 61 ORFs, 28 of which show similarities to known proteins with assigned functions (Fig. 1).

Surprisingly, pE88 was found to encode an additional virulence factor, a 114 kDa collagenase, which we designated ColT (CTP33). Said enzyme plays an important role in *C. tetani* pathogenesis because of its activity to destroy tissue integrity in the infected host. A multiple sequence alignment with other known clostridial collagenases of *C. perfringens* (Matsushita, O., et al., (1994) *J. Bacteriol.* 176, 149-156), *C. histolyticum* (Matsushita, O., et al., (1999) *J. Bacteriol.* 181, 923-933) and the one encoded on the genome of *C. botulinum* (Sanger Centre, *C. botulinum* sequencing project; online access: ftp://ftp.sanger.ac.uk/pub/pathogens/cb/), designated ColB, revealed a different domain structure: ColT lacks segment 2, the so-called PKD domain of unknown function (Fig. 2). Among clostridial collagenases ColT shows the strongest similarity to ColB of *C. botulinum* with 79% amino acid (aa) identity over segment 1.

Accordingly, in a preferred embodiment, the invention is directed to a *C. tetani* collagenase comprising a catalytic domain and a collagen-binding domain.

*C. tetani* collagenase specifically cleaves the x-glycine-binding within the motif proline-X-proline of collagen, wherein X is a neutral amino acid.

The lack of the PKD domain and the presence of only a single collagenase within *C. tetani* point to a broader substrate specificity of the collagenase according to the present invention compared to commercially available *C. histolyticum* collagenases. The latter are characterized by a more narrow substrate specificity. Accordingly, the novel collagenase presented herein may advantageously be used in a broader range of applications.

In a first aspect, said broader substrate specificity is supported by the lack of the PKD domain. Said domain was shown to be responsible for the modulation of protein-protein interactions (Tsiokas L., et al., *(1997), Proc Natl Acad Sci U S A Jun 24;94(13):6965-70).* In the case of *C. tetani* collagenase, it is expected that the PKD domain would also regulate the substrate specificity between the enzyme (which is a protein) and substrate (which is also protein). Accordingly, the lack of a domain which is normally involved in protein-protein interaction points to the fact that the present *C. tetani* collagenase displays a broader substrate specificity.

In a second aspect, we revealed the surprising fact that *C. tetani* harbours only one collagenase, whereas in *C. histolyticum,* for example, at least 2 different collagenase and 4 additional *gelatinases* (gelatine corresponds to denatured collagen) are present (Matsushita et al., *J. Bact.* 181 No. 3, 1999, pp 923-933). Said enzymes display quite different hydrolytic activities, which may also complement each other. Accordingly, the presence of only one collagenase in *C. tetani* leads to the conclusion that said collagenase is characterized by a broader substrate specificity.

The possible function of the PKD domain present in other clostridial collagenases was analyzed by generating deletion mutants of *C. histolyticum* collagenase (Matsushita O., et al., 1998, *J. Biol. Chem.* Vol. 273 (6), pp. 3643-3648). According to said publication, the PKD domain itself does not show any binding activity. However, upon addition of the PKD domain to the collagen-binding domain, binding affinity of said mutant was increased close to that of the full-length enzyme. Consequently, the authors hypothesized that the PKD domain assists proper folding of the minimal collagen-binding domain.

The amino acid sequence which represents the collagenase according to the present invention is represented by SEQ ID NO.1 (see also Figure 6). The *C. tetani* collagenase is characterized by an amino acid sequence of 991 amino acids with a calculated molecular weight of 114,375 kDa and an isoelectric point of pH = 6,12 (of the propeptide). *C. tetani* collagenase is translated as a propeptide and is cleaved upon extracellular transport by a signal peptidase in order to yield the mature protein.

Furthermore, the invention is directed to an isolated nucleic acid sequence characterized by SEQ ID NO.2, which encodes the *C. tetani* collagenase. Said nucleic acid sequence is 2967 bp long (see also Figure 5).

### Production of the collagenase derived from C. tetani

The present invention provides for the first time an isolated nucleic acid sequence which encodes a collagenase derived from *C. tetani.* Accordingly, the availability of said nucleic acid sequence enables the recombinant production of the *C. tetani* collagenase in host cells transformed with expression vectors comprising DNA encoding the collagenase. Subsequently, the expressed collagenase may be isolated from host cells.

For the production of C. tetani collagenase, prokaryotic host cells may be used, preferably *E. coli* host cells. In a preferred embodiment, the Rosetta^{TM} *E. coli* strain is used.

Rosetta^{TM} Rosetta host strains (supplied by Novagen, Cat. No. 70949) are Tuner^{TM} derivatives designed to enhance the expression of eukaryotic proteins that contain codons rarely used in *E. coli.* These strains supply tRNAs for AGG, AGA, AUA, CUA, CCC, GGA codons on a compatible chloramphenicol-resistant plasmid. Thus the Rosetta strains provide for "universal" translation which is otherwise limited by the codon usage of *E. coli.* The tRNA genes are driven by their native promoters. In Rosetta(DE3)pLysS and Rosetta(DE3)pLacI, the rare tRNA genes are present on the same plasmids that carry the T7 lysozyme and *lac* repressor genes, respectively.

Apart from prokaryotic microorganisms, production of collagenase may also be accomplished in eukaryotic host cells (for example CHO cells, yeast or insect cells). Preferably, yeast or insect cells are used as eukaryotic host cells. Regarding yeast host cells, *S. cerevisiae* is the most commonly used eukaryotic microorganism, although a number of other strains is also available.

Once an appropriate host cell is selected, said host cell is transformed with an expression vector comprising the nucleic acid sequence of interest.

"Transformation" of host cells denotes a method for introducing DNA into an organism so that the DNA is replicable, either as an extrachromosomal element or by chromosomal integration. The DNA to be introduced is comprised within an expression vector.

The term "expression vector" denotes a cloning vector, which, after introduction into the appropriate host cell, enables the transcription and translation of the exogenous gene cloned into said vector. Construction of suitable expression vectors containing the desired coding and control sequences employ standard ligation techniques.

A useful expression vector for the invention is a replicable DNA construct in which a DNA sequence encoding *C. tetani* collagenase (or an analogue or variant thereof) is operatively linked to suitable control sequences capable of effecting the expression of collagenase (or of the collagenase analogue or variant) in a suitable host. Such control sequences include a transcriptional promoter, an optional operator sequence to control transcription, a sequence encoding suitable mRNA ribosomal binding sites, and sequences which control termination of transcription and translation. The vector preferably also comprises a selectable marker.

According to the present invention, the bacterial expression system pET 22b (Novagen, Cat No. 69774-3) was used. Said vector carries an N-terminal pelB signal sequence for a potential periplasmic localization in *E. coli* as well as an optional C terminal His tag for easy affinity purification of the recombinant polypeptide.

Another approved bacterial expression system is the pASK-IBA2 vector from IBA (Göttingen). This vector carries a N terminal signal sequence for a potential periplasmic localization and a C terminal *Strep*-Tag for easy affinity purification on Streptactin-based chromatographic columns.

Once the expression vector comprising the nucleic acid sequence of interest has been prepared, it may be introduced into an appropriate host cell by any of a variety of suitable methods: transformation, transfection, conjugation, protoplast fusion, electroporation, calcium phosphate precipitation, direct microinjection etc., which are well known to the person of ordinary skill in the art.

After introduction of the vector, recipient cells are grown in a selective medium, which selects for the growth of vector-containing cells. Expression of the cloned gene sequence results in the production of the collagenase. The expressed protein may be isolated and purified in accordance with conventional methods, such as extraction, precipitation, chromatography, affinity chromatography, electrophoresis or the like. The recited techniques are well known to the person skilled in the art.

### Preparation of collagenase variants

The present invention is also directed to variants of the collagenase as represented by the amino acid sequence of SEQ ID NO: 1. Said amino acid sequence variants may be substitutional, insertional or deletion variants.

Insertional variants include amino and/or carboxyterminal fusions as well as intrasequence insertions of single or multiple amino acid residues. Typically, an insertional variant comprises an addition of 1-14, preferably of 1-7 and particularly preferred of 1-5 amino acids to the sequence represented by SEQ ID NO:1. Preferred fusions are aminoterminal fusions with either prokaryotic peptides or signal peptides of prokaryotic, yeast, viral or host cell signal sequences.

Deletion variants are characterized by the removal of one or more amino acid residues from the collagenase protein. Typically, no more than 2-6 residues will be deleted at any site within the collagenase protein, although deletion of larger fragments may be possible, as long as the biological activity of collagenase is retained. In one embodiment of the invention, a deletion variant comprises a deletion of 1-5 amino acids, preferably of 1-3 amino acids.

"Biological activity of collagenase" refers to the property of *C. tetani* collagenase to specifically cleave the x-glycine-binding within the motif proline-X-proline of collagen, wherein X is a neutral amino acid.

Substitutional variants are those in which at least one residue in SEQ ID NO.1 has been removed and a different residue inserted in its place. In general, amino acids are substituted by amino acids with similar properties as regards hydrophobicity, hydrophilicity, electronegativity, side chains and the like. In an embodiment of the invention, the term "substitutional variant" denotes a substitution of 1 to 9, preferably 1-7 und particularly preferred of 1-5 amino acid residues in the amino acid sequence of SEQ ID NO: 1.

Usually, substitutions of amino acids merely concern a single amino acid residue, whereas insertions refer to a region of 1-10 amino acid residues. Deletional variants typically refer to a region of 1-12 amino acid residues.

The term "variants" also includes an amino- or carboxyterminal truncation of amino acid sequence SEQ ID NO:1 by 1-3, preferably 1-2 and particularly preferred by 1 amino acid. Moreover, post-translational modifications like glycosylation, acetylation, phosphorylation and the like are also comprised within the term "variants".

The above-recited variants are typically prepared by known techniques of site specific mutagenesis of nucleotides in the nucleic acid sequence encoding the collagenase, thereby producing DNA encoding the variant, and thereafter expressing the DNA in recombinant cell culture. The variants typically exhibit the same biological activity as the naturally-occuring collagenase, although variants are also selected in order to modify the structural or functional characteristics of *C. tetani* collagenase.

As a starting material for the preparation of said variants, the isolated nucleic acid sequence according to SEQ ID NO:2 may be used. Thus, the invention is also directed to isolated nucleic acid sequences which encode variants of the naturally occurring collagenase. Furthermore, naturally occurring allelic variants of the *C. tetani* collagenase are also comprised within the scope of the invention.

In addition, the invention also comprises analogues of the collagenase as represented by SEQ ID NO:1. The term "analogue", as used in the context of the present invention denotes an amino acid sequence which displays 40-95 %, preferably 50-90 % and more preferably 70-90 % homology to the amino acid sequence of SEQ ID NO: 1.

The biological activity of the *C. tetani* collagenase, its analogues and variants of the present invention can be analysed by various methods.

In a first aspect, the collagenase activity can be recorded by using the convenient spectrophotometric assay based on the hydrolysis of the artificial 2-furfuranacrylolyl-Leu-Gly-Pro-Ala (FALGPA) peptide. Apparently, there is sufficient structural similarity between the 2-furfuranacrylolyl group and the proline in collagen to be an exellent substrate for collagenases. Assays of this type are made possible by the blue shift in adsorption when the peptide bond between the first and the second residue is hydrolysed by the activity of collagenase. Therefore, the FALGPA concentration can be monitored in the spectrophotometer using an ε₃₀₅ value of 24.7 M⁻¹ cm ⁻¹ (van der Wart and Steinbrink, *Analyt. Biochemistry* 113 (1981), pp. 356-365)

A second method suitable for use in the present invention comprises zymography using native gel electrophoresis in gelatin substrate gels. After staining with Coomassie brilliant blue, protein bands with hydrolytic activity were detected as clear zones in the blue background.

### Collagenase antibodies

The present invention is also directed to poly- or monoclonal antibodies directed against the collagenase characterized by the amino acid sequence of SEQ ID NO: 1 or against a variant or analogue thereof.

Said antibodies may be obtained by the use of the protein characterized by SEQ ID NO.1 or a variant or analogue thereof as antigen. Polyclonal antibodies may be obtained by injection of the respective protein into an animal, for example a rabbit, and by recovering serum from said immunized animals.

Furthermore, monoclonal antibodies are also comprised within the scope of the present invention. The preparation of hybridoma cell lines for the production of said monoclonal antibodies against the protein is achieved by fusion of an immortal cell line with antibody-producing lymphocytes derived from the species immunized with the respective protein. This may be achieved by methods known to the person skilled in the art.

Fused cells may then be separated from the immortal cells by well known methods, which may comprise a selection process of the fused cells for expression of Hypoxanthine-guanine-phosphoribosyltransferase (HGPRT). While immortal cells lack a functional HGPRT gene and hence, are not able to grow on aminopterin-containing media, hybridoma cells, which contain the HGPRT gene of the lymphocytes, will grow. Fused cells are then assayed for the presence of antibodies against the collagenase represented by amino acid sequence SEQ ID NO: 1 or variants or analogues thereof.

### Use of the C. tetani collagenase

The present invention is also directed to the use of the collagenase as characterized by SEQ ID NO: 1, analogues and variants thereof for cell culture and therapeutic purposes.

Bacterial collagenases have already been shown to be particularly suitable for the isolation of viable cells from tissue. Collagenases are especially convenient when tissues are too fibrous or too sensitive to allow the use of trypsin, which is ineffective on fibrous material, and damaging to sensitive material. Furthermore, collagenases can be used for the isolation of viable pancreatic islets from different species, for the isolation of cardiomyocytes suitable for the preparation of cultures and for the isolation of hepatocytes, which are used in tumor promotion and initiation studies, for studying cellular control mechanisms and in drug and carcinogen assay systems. Collagenases may also be used for the isolation of cells from bone, thyroid glands, ovarian and uterine tissues. Disintegration of the above-recited tissues can be achieved either by perfusing whole organs, or by incubating smaller pieces of tissue with enzyme solution.

Accordingly, one embodiment of the invention is directed to the use of *C. tetani* collagenase for the use of isolation of viable cells from tissue, preferably to the isolation of viable pancreatic islets, cardiomyoctes, hepatocytes, cells from bone, thyroid glands, and ovarian and uterine tissues. The respective tissues may be of human or animal origin.

Furthermore, the collagenase according to the present invention may also be used for therapeutic purposes in the treatment of diseases associated with excessive or abnormal collagen deposition.

Collagens are the major protein constituents of the extracellular matrix and the most abundant proteins in all higher organisms (Mayne R., and Burgeson, R.E., 1987, *Structure and Function of Collagen types,* Academic Press, Orlando, FL). The tightly coiled triple helical collagen molecule assembles into water-insoluble fibers or sheets which are cleaved only by collagenases, and which are resistant to other proteases.

There are many disease states which involve an excessive deposition of collagen. Examples thereof include Dupuytren's disease, Peyronie's disease and glaucoma, which are mostly treated by surgery. The possibility of the use of *C. histolyticum* collagenase for the treatment of said diseases has already been analysed (Gelbard M.K., et al., 1993, *J. Urol.* 149:56-58; Starkweather, K.D., 1996, *J. Hand Surg.* 21:490-495).

Accordingly, another embodiment of the invention is directed to the use of *C. tetani* collagenase for the treatment of diseases associated with excessive collagen deposition, in particular glaucoma, Dupuytren's disease and Peyronie's disease.

Apart from the use for the treatment of diseases associated with an excessive deposition of collagen matrix, the *C. tetani* collagenase may also be used for the treatment of various injuries of any substrate having a structure rich in collagen. Examples of such injuries include burn, ulcer, scab, white hard scab of collagen base, cheloid, necrosis, particularly necrosis by decubitus or ulcer, and the like.

Furthermore, collagenases have been shown to be useful enzymes in wound healing, since they significantly enhance cell growth and migration after injury. Accordingly, the collagenase according to the present invention is also intended to be used as an agent in wound healing.

In a further embodiment, the invention is also directed to the use of the *C. tetani* collagenase, analogues and variants thereof for the preparation of pharmaceutical compositions for the treatment of the above-recited diseases and conditions. Pharmaceutical compositions may also comprise suitable carrier molecules or excipients.

In order to enhance the collagenolytic activity, the *C. tetani* collagenase may also be used in conjunction with other enzymes, such as proteases (for example serine proteases like trypsin, chymotrypsin), cysteine proteases, elastases, hyaluronidases, lipases and the like.

### Examples

### Example 1: Sequence Analysis of the C. tetani genome

1. *Sequencing strategy.* Genomic DNA of *C. tetani* E88, a variant of the strain Massachusetts used in vaccine production, was extracted and sheared randomly. Several shotgun libraries were constructed using size fractions ranging from 1 to 3 kb. A cosmid library was constructed from Sau3AI partially digested genomic DNA cloned in the cosmid vector SuperCos1 (Stratagene Inc.). Insert ends of the recombinant plasmids and cosmids were sequenced using dye-terminator chemistry with MegaBACE 1000 and ABI Prism 377 DNA automated sequencers. Sequences were processed with Phred and assembled into contigs using the Phrap assembling tool (Ewing, B., et al., (1998) *Genome Res.* 8, 175-185). Sequence editing was done using GAP4, which is part of the Staden package software (Staden, R., Beal, K. F. & Bonfield, J. K. (2000) *Methods Mol. Biol.* 132, 115-130). A coverage of 9.2-fold was obtained after the assembly of 44500 sequences. In order to solve problems with misassembled regions caused by repetitive sequences and to close remaining sequence gaps, PCR-based techniques and primer walking on recombinant plasmids and cosmids were applied.
2. *ORF prediction and annotation.* Initial open reading frame (ORF) prediction was accomplished using the Glimmer program (http://www.tigr.org/software/glimmer/). The result was verified and improved manually using criteria such as the presence of a ribosome binding site and codon usage analysis. Annotation was done first automatically by the ERGO annotation tool (Integrated Genomics, Inc., http://www.integratedgenomics.com), which was verified and refined by searching derived protein sequences against the public GenBank/EMBL databases using the Blast program (Altschul, S. F., et al., (1990) *J. Mol. Biol.* 215, 403-410).
3. *Comparative genomics.* For comparative analysis each ORF of *C. tetani* was searched against all ORFs of *C*. *perfringens* and *C. acetobutylicum* using the BLAST program. Significant homology was defined as >30% amino acid identity over >60% of both the *C. tetani* protein sequence and the homologous sequence of *C. perfringens* or *C. acetobutylicum,* respectively.
4. *General features of the C. tetani genome.* The chromosome of *C. tetani* contains 2,799,250 bp with a G+C content of 28.6%. The 74,082 bp plasmid pE88 exhibits a remarkably low G+C content of 24.5%. The replication origin of the chromosome was identified on the basis of GC-skew analysis, the distinctive inflection point in the coding strand and the vicinal presence of characteristic replication proteins such as DnaA (Fig. 1, see also supporting information). 82% of the 2,368 predicted ORFs on the chromosome are transcribed in the same direction as DNA replication. A similar figure (83%) was calculated from the sequence data of *C. perfringens* (Shimizu, T., et al., (2002) *Proc. Natl Acad. Sci. USA* 99, 996-1001). This distinctive co-directionality of replication and transcription is not seen in the genomes of pathogens such as *Vibrio cholerae* or *Yersinia pestis* or in archaeal genomes such as *Methanosarcina mazei* (Heidelberg, J. F., et al., (2000) *Nature* 406, 477-483; Parkhill, J., et al., (2001) *Nature* 413, 523-527; Deppenmeier, U., et al., (2002) *J. Mol. Microbiol. Biotechnol.* 4, 453-461).
Only a few mobile elements could be identified in the genome of *C. tetani:* 16 transposase genes are present, which can be classified in 4 different insertion sequence families. Most of these genes seem to be non-functional because of insertions, deletions and point mutations resulting in frame-shifted or degenerated ORFs. G+C variation within the genome is very low; the only regions with a significantly higher G+C content (approx. 50%) are those harboring the six rRNA gene clusters and genes encoding ribosomal proteins. This lack of G+C fluctuation indicates that recent events of gene acquisition by lateral gene transfer have not taken place and that the *C. tetani* genome is more stable than the genomes of enteropathogens.
5. *The tetanus toxin-encoding plasmid pE88.* The 74 kb *C. tetani* plasmid pE88 harbors the genes for the tetanus toxin (*tet*X, CTP60) and its direct transcriptional regulator TetR (Eisel, U., et al., (1986) *EMBO* J 5, 2495-2502; Fairweather, N. F. & Lyness, V. A. (1986) *Nucleic Acids Res.* 14, 7809-7812; Marvaud, J.-C., et al., (1998) *Infect. Immun.* 66, 5698-5702; Finn, C. W., et al., (1984) *Science* 224, 881-884).
We identified 61 ORFs, 28 of which show similarities to known proteins with assigned functions (Fig. 1). pE88 encodes an additional virulence factor, a 114 kDa collagenase, designated ColT (CTP33), an enzyme which plays an important role in *C. tetani* pathogenesis because of its activity to destroy tissue integrity in the infected host (Harrington, D. J. (1996) *Infect. Immun.* 64, 1885-1891). A multiple sequence alignment with other known clostridial collagenases of *C. perfringens* (Matsushita, O., et al., (1994) *J. Bacteriol.* 176, 149-156), *C. histolyticum* (Matsushita, O., et al., (1999) *J. Bacteriol.* 181, 923-933) and the one encoded on the genome of *C. botulinum* (Sanger Centre, *C. botulinum* sequencing project; online access: ftp://ftp.sanger.ac.uk/pub/pathogens/cb/), designated ColB, revealed a different domain structure: ColT lacks segment 2, the so-called PKD domain of unknown function (Fig. 2). Among clostridial collagenases ColT shows the strongest similarity to ColB of *C. botulinum* with 79% amino acid (aa) identity over segment 1.
Furthermore, nine transport-related genes similar to ABC transporters or associated permeases are encoded on pE88 as well as eight further hypothetical genes with multiple transmembrane helices, which indicates their involvement in transport or signal transduction.
Of special interest for an understanding of the regulatory network governing the tetanus toxin formation are seven regulatory proteins on pE88. Among them is a two-component system (CTP21/22), which shows high similarities to systems of other Gram-positive bacteria such as *Bacillus cereus, B. halodurans* and *C. botulinum.*
Further regulatory proteins, including TetR, are summarized in Table 1, *infra.* They show some similarity to each other, as well as to TxeR from *C. difficile,* the alternative sigma factor regulating the synthesis of the toxins ToxA and ToxB (Mani, N. & Dupuy, B. (2001) *Proc. Natl Acad. Sci. USA* 98, 5844-5849), and to UviA of *C.* *perfringens,* a putative positive regulator of the UV-inducible bacteriocin gene (Garnier, T. & Cole, S. T. (1988) *Mol. Microbiol.* 2, 607-614). CTP05 seems to be a pseudogene disrupted by an authentic frameshift.

**Table 1:**

| Sequence similarities of regulatory elements encoded on pE88 to characterized regulatory proteins of *C. perfringens, C. difficile* and *C. botulinum.* | | | | |
|---|---|---|---|---|
| ORF name and size (aa) * | Annotation | UviA (185 aa) *C. perfringens* | TxeR (184 aa) *C. difficile* | BotR/A (178 aa) *C. botulinum* |
| CTP5 | Frameshift, | 32% (48%) | 31% (49%) | 26% (43%) |
| (172 aa) | UviA homolog | over 185 aa | over 170 aa | over 178 aa |
| | | | | |
| CTP10 (179 aa) | putative sigmafactor / DNA binding protein | 24% (43%) over 184 aa | 28% (47%) over 82 aa | not significant |
| | | | | |
| CTP11 (184 aa) | putative sigmafactor / DNA binding protein | 25% (44%) over 180 aa | 24% (44%) over 179 aa | not significant |
| | | | | |
| CTP61 | positive | 27% (49%) | 26% (47%) | 67% (85%) |
| (TetR) | regulator of *tet*X | over 183 aa | over 184 aa | over 177 aa |
| 177 aa | | | | |

| | | | | |
|---|---|---|---|---|
| Percentages indicate identical (in brackets: similar) amino acids. * Not listed here is CTP4 (71 aa) showing homology only to UviB (64 aa) of *C. perfringens* (40% identity over 67 aa) and the two-component system CTP21/22 | | | | |

The origin of pE88 remains unclear. Over 50% of all ORFs on pE88 are unique to C. *tetani.* A mobile element, present in two copies, shows strongest similarity to a transposase of *B. cereus* (60% identity over 355 aa). The putative replication protein of pE88 is duplicated (CTP1 and CTP45). It shows homology only to a protein (40% identity over 438 aa) encoded on the plasmid of the alkaliphilic *Bacillus* strain KSM-KP43, and to the replication protein (39% identity over 418 aa) on pIP404, the bacteriocin- and UviA-encoding 10 kb plasmid of *C. perfringens* CPN50 (Garnier, T. & Cole, S. T. (1988) *Plasmid* 19, 151-160).
6. *Virulence factors on the chromosome.* In addition to pE88, the *C. tetani* chromosome was searched for virulence genes. The results of said analysis are summarized in Table 2, *infra.*

**Table 2:**

| Virulence-related genes in *C. tetani.* | | | |
|---|---|---|---|
| **ORF number and size (aa)** | **Product** | **Similar sequences** | **Comment** |
| CTP60(1315) *tet*X CTP33 (991) *col*T | Tetanus toxin Collagenase | Botulinum toxin, Cb Cp, Ch, Cb | unique in C. *tetani* and Cb different domain structure of ColT (Fig. 2) |
| CTC164 (224), CTC471 (642), CTC1606 (577) | Fibronectin-binding protein | Cp, Cb, Cd, Lm, Li | enables bacteria to colonize wound tissues and blood clots |
| CTC594 (291), CTC1503 (317) | Platelet activating factor acetylhydrolase (PAF-AH) | *Streptococcus pneumoniae, S. mutans, S. pyogenes* | PAF-AH inactivates platelet-activating factor through cleavage of an acetyl group |
| CTC586 (211) CTC 1574 (272) | Hemolysin III Hemolysin | Cp, Cb, Ca Cp, Cb, Ca, Cd | predicted membrane protein predicted rRNA methylase |
| CTC1855 (533) | Myosin-crossreactive antigen | Cp, Lm, Li, Cb, *S. pyogenes* | similar with class II major histocompatibility antigens |
| CTC1888 (527) | Tetanolysin O | Perfringolysin O (Cp), *S*. *Pyogenes,* Lm | cytolysin, catalyzing the lysis of cholesterol-containing membranes |
| CTC2255 (518), CTC2261 (514) ^{*a*}, CTC462 | Virulence factor MVIN | Cb, Ca, Cd | membrane protein |
| (1080), CTC491 (508) CTC504 (592) | Cwp66 homologue | Cd (28 homologues), Cb (8 homologues) | SLP with adhesive properties, mediates adherence to host tissue |
| CTC494 (706), CTC495 (695), CTC520 (871) | Internalin A homologue | Lm, Cb, Ca | Intemalin A mediates the entry of *Listeria* into host cells |
| ^{*b*}, CTC465 | | | |
| (1334), CTC747 (1416), CTC749 (1900) | Surface layer and/or or adhesion protein | | most of them contain the Big2-domain, which is found in surface proteins such as intimin |

| | | | |
|---|---|---|---|
| a) additional ORFs: CTC520 (871), CTC521 (487), CTC523 (354), CTC691 (505), CTC696 (518), CTC748 (462), CTC774 (667), CTC775 (353), CTC1202 (492), CTC1364 (648), CTC1872 (471), CTC1980 (722), CTC2092 (508), CTC2093 (708), CTC515 (584), CTC518 (585). | | | |
| b) additional ORFs: CTC767 (528), CTC769 (568), CTC770 (603), CTC771 (374), CTC772 (360), CTC776 (355), CTC750 (1511), CTC777 (1880). | | | |

Ca, *C. acetobutylicum;* Cb, *C. botulinum;* Cd, *C. difficile;* Ch, *C. histolyticum;* Cp, *C. perfringens;* Li, *Listeria innocua;* Lm, *L. monocytogenes.*
ORFs for hemolysins, tetanolysin O and fibronectin-binding proteins were detected exhibiting homology to ORFs in other clostridial species including *C. perfringens* (Shimizu, T., et al., (2002) *Proc. Natl Acad. Sci. USA* 99, 996-1001; Rood, J. I. (1998) *Annu. Rev. Microbiol.* 52, 333-360).
A difference to *C. perfringens* is the presence of an array of surface layer proteins (SLPs) in *C. tetani,* all of which are absent in *C. perfringens:* 19 homologues of Cwp66, an adhesin of *C. difficile* were found (Waligora, A. J., et al., (2001) *Infect. Immun.* 69, 2144-2153; Calabi, E. & Fairweather, N. F. (2002) *J. Bacteriol.* 184, 3886-3897). In addition, 11 SLP-related ORFs were identified, which have no homologues in other sequenced clostridial genomes. All these proteins, as well as the Cwp66 homologues, possess a characteristic leader peptide suggesting that these proteins are exported. The genes of the SLPs were found to be clustered; otherwise pathogenicity islands or mobile elements in flanking regions of virulence genes could not be detected.
7. *Comparative genomics.* The genome of *C. tetani* was compared with the genomes of both *C. perfringens* (Shimizu, T., et al., (2002) *Proc. Natl Acad. Sci. USA* 99, 996-1001), and the non-pathogenic *C. acetobutylicum* (Nölling, J., et al., (2001) *J. Bacteriol.* 183, 4823-4838), in order to analyse which other gene products could contribute to the pathogenic life style of *C. tetani.* The results are summarized in Figure 3.
Homologous ORFs were identified. Focusing on *C. tetani,* four groups of ORFs could be distinguished (Fig. 3). Two of them are of special interest, the 199 ORFs which are common to both *C. tetani* and *C. perfringens,* and the 516 ORFs of *C. tetani* which have no homologues in *C. perfringens* and *C. acetobutylicum* (see supporting information for the complete ORF lists of these groups). Remarkably, ORFs encoding 27 peptidases were found within these two groups, many of them being predicted zinc-metalloproteases. 15 ORFs for ethanolamine utilization similar to the *eut* genes of the genera *Salmonella* (Kofoid, E., et al., (1999) *J. Bacteriol.* 181, 5317-5329) and *Listeria* were detected as well as 21 ORFs for amino acid decomposition including glutamate mutase, tryptophanase, histidine ammonia lyase and lysine-2,3-aminomutase.
Furthermore, we revealed the striking presence of 35 genes for sodium ion-dependent systems (Fig. 4). The primary source for the ion-motive force at the cytoplasmic membrane of *C. tetani* is a vacuolar(V)-type ATPase similar to the ATPase of *Enterococcus hirae* which is a Na⁺ pump (Kakinuma, Y. & Igarashi, K. (1995) *FEBS Lett.* 359, 255-258). *C. tetani* does not harbour the genes of the F₀F₁-type ATPase; these are present in *C. acetobutylicum* and *C. perfringens.* The lack of the latter genes in *C. tetani* is highly exceptional.
Therefore, part of the ATP synthesized in the course of the fermentative metabolism of *C. tetani* is hydrolysed by the V-type ATPase to generate a Na⁺-motive force at the membrane. This force is used to drive various transport processes: several sodium-dependent substrate cotransporters are present and at least six of the multidrug resistance exporters seem to be driven by Na⁺-intrusion, while there are apparently no proton-dependent systems present. As a common coupling device between proton and sodium ion gradients several H⁺/Na⁺ antiporters were detected, challenging the search for a primary proton pump: a proton-translocating pyrophosphatase showing similarity to systems in plant vacuoles is present in *C. tetani* but absent in other clostridia. Furthermore, the genes for a membrane-bound electron transport chain could be identified in the genomes of *C. tetani* and *C. perfringens.* This system shows strong homology to the RNF (*Rhodobacter* specific nitrogen fixation) system of *Rhodobacter capsulatus* as well as to the NQR system found in many aerobic pathogens such as *Vibrio cholerae, S. typhimurium, Yersinia pestis* and *Haemophilus influenzae,* in which it apparently functions as a Na⁺-translocating NADH dehydrogenase (Häse, C. C., et al., (2001) *Microbiol. Mol. Biol. Rev.* 65, 353-370; Schmehl, M., et al., (1993) *Mol. Gen. Genet.* 241, 602-615).
As there are no reports for quinones in clostridia, such a NADH dehydrogenase could be involved in the fermentation of amino acids by *C. tetani* as indicated in Fig. 4. A highly active NADH dehydrogenase was detected in washed membranes of the closely related *C. tetanomorphum* (W. Buckel, Germany, personal communication). This may be the system which is missing for an understanding of electron flow from reduced ferredoxin, generated in the pyruvate:ferredoxin oxidoreductase reaction, via NADH to the NADH-consuming dehydrogenases of the butyrate pathway.

### Example 2: Expression of C. tetani collagenase

A 3.0 kb oligonucleotide containing the *C. tetani* collagenase was constructed as follows. A truncated oligonucleotide without the *C. tetani* specific signal sequence was amplified by polymerase chain reaction using appropriate primers containing Xhol and Ncol cleavage sites. The amplified fragment was cloned into the pET 22b vector (Novagen, Cat No. 69774-3). This vector carries an N terminal pelB signal sequence for a potential periplasmic localization in *E. coli* as well as an optional C terminal His tag for easy affinity purification of the recombinant polypeptide. This construct was transformed into *E. coli* Rosetta^{TM} host strains (supplied by Novagen, Cat. No. 70949) using electroporation. Expression was induced by IPTG, the culture supernatant was subjected to an affinity purification by means of a Ni-NTA chromatographic column.

The collagenase activity was recorded by using the convenient spectrophotometric assay based on the hydrolysis of the artificial 2-furfuranacrylolyl-Leu-Gly-Pro-Ala (FALGPA) peptide. Apparently, there is sufficient structural similarity between the 2-furfuranacrylolyl group and the proline in collagen to be an excellent substrate for collagenases. Assays of this type are made possible by the blue shift in adsorption when the peptide bond between the first and the second residue is hydrolysed by the activity of collagenase. Therefore, the FALGPA concentration can be monitored in the spectrophotometer using an ε₃₀₅ value of 24.7 M⁻¹ cm ⁻¹ (van der Wart and Steinbrink, *Analyt. Biochemistry* 113 (1981), pp. 356-365).

The tests were carried out in 50 mM Tricine buffer, pH 7.5, 0.4 M NaCl, with FALGPA concentrations between 0.05 mM and 0.15 mM.

A similar test based on the phenylazobenzyloxycarbonyl-Leu-Gly-Pro-Arg peptide (pz peptide) in a photometric test system may also be employed.

As a further method, zymography using native gel electrophoresis in gelatin substrate gels was used. After staining with Coomassie brilliant blue, protein bands with hydrolytic activity were detected as clear zones in the blue background.

### Example 3: Analysis of collagenase protein secretion

*Protein secretion.* Only little is known about protein secretory systems in Clostridia. Until now, it is not understood how the tetanus toxin is exported. As protein secretion is an important part in establishing a pathogenic phenotype, it is worth having a closer look on secretory systems and secreted proteins of *C. tetani.*

A characteristic signal peptide, recognised by the Sec-dependent secretory mechanism, could be detected in 419 deduced proteins of *C. tetani.* Thereof, 101 proteins are involved in transport, inclusively periplasmic substrate-binding proteins. About 160 proteins are conserved proteins of unknown function, most of them strongly hydrophobic. Virulence and surface proteins, peptidases, sporulation-specific proteins as well as sensory proteins like sensor histidine kinases and chemotaxis proteins have a predicted N-terminal signal peptide. Thus, the well-studied prokaryotic Sec-system (Driessen, A. J., Fekkes, P. & van der Wolk, J. P. (1998) *Curr Opin Microbiol* 1, 216-222) is apparently the major protein translocation apparatus in *C. tetani.* However, not all *sec* genes of *Escherichia coli* and related Gram-negative species are present in the genomes of *C. tetani* and other clostridia. SecA, SecD, SecE, SecF and SecY could be identified; SecB, a translocation-specific chaperone and SecG, usually part of the Sec YEG integral membrane complex seem to be missing in the clostridial group, indicating the existence of a modified Sec-system.

Alternatively, other proteins involved in protein secretion may take over the SecBG functions. A homologue of YajC (CTG2208) is present, which can form an integral membrane heterotrimeric complex together with SecD and SecF (Nouwen, N. & Driessen, A. J. (2002) *Mol Microbiol* 44, 1397-1405). A protein in the immediate vicinity of the putative *ori* (CTG0096) exhibits some homology to YidC of *E. coli,* which has recently been shown to interact with the SecDFyajC complex (Nouwen, N. & Driessen, A. J. (2002) *Mol Microbiol* 44, 1397-1405). Furthermore, CTG166 and CTG458, present exclusively in clostridia, show homologies to the C-terminal domain of SecA.

In additional, *C. tetani* possesses genes of a signal recognition particle (SRP)-like pathway, a protein translocation system similar to mammalian SRP. This system is thought to play an essential role in protein translocation and membrane protein insertion (Lutcke, H. (1995) *Eur. J. Biochem.* 228, 531-550; Zwieb, C. & Samuelsson, T. (2000) *Nucleic Acids Res.* 28, 171-172). It consists of the SRP54 homologue Ffh (CTG1249) and a small cytoplasmic RNA (upstream of CTG0075), together forming the SRP, which binds to the signal sequence when it emerges from the ribosomes and FtsY (CTG1246), the alpha subunit of the SRP receptor, which is thought to mediate the reception and insertion of a subset of proteins at the cytoplasmic membrane.

Whether Ffh and FtsY are part of an independent protein secretion pathway or whether these proteins take over functions of the Sec-dependent pathway, as proposed previously, has to be elucidated (van Wely, K. H., et al., (2001) *FEMS Microbiol Rev* 25, 437-454; Kunst, F., et al., (1997) *Nature* 390, 249-256).

In *C. tetani* as well as in all other sequenced clostridia there are genes clustered with FtsY and Ffh, which are putatively involved in the SRP-dependent secretory mechanism: a signal peptidase (CTG1253), a ribonuclease (CTG1255), a GTP-binding protein (CTG1254), a 16S rRNA processing protein (CTG1250), two ribosomal proteins (L19P and S16P), a tRNA (Guanine-N1)-methyltransferase (CTG1251) and a RNA-binding protein (CTG1248).

The Sec-independent TAT (twin arginine translocation) system is absent from the C. *tetani* genome and all clostridial genomes known so far.

The finding that some of the virulence factors, like the tetanus toxin, two fibronectin-binding proteins and hemolysin, lack a typical signal peptide, indicates the presence of an alternative, so far unknown mechanism for toxin export. This was also postulated for the export of toxA and toxB of *C. difficile* (Mukherjee, K., et al., (2002) *Microbiology* 148, 2245-2253.).

*Conclusions.* It can be concluded that *C. tetani* is an invasive anaerobic pathogen possessing a wide arsenal of virulence factors; this arsenal is exacerbated by the ability to produce the tetanus toxin and possibly by the presence of various surface layer proteins of yet unknown function. The performance of an extensive sodium ion bioenergetics might be an additional factor for the successful invasion of infected tissue. The genome sequence of *C. tetani* now provides the information on regulatory systems so that the signal transduction pathway leading to tetanus toxin expression can be studied.

## Claims

1. A collagenase derived from a bacterium of the species *Clostridium tetani.*

2. The collagenase according to claim 1, wherein said collagenase comprises a catalytic domain and a collagen-binding domain.

3. The collagenase according to claim 1, wherein said collagenase is represented by the amino acid sequence of SEQ ID NO: 1 or by analogues or variants thereof.

4. The collagenase according to claim 3, wherein said analogues are **characterized by** amino acid sequences with 70-90 % homology to the amino acid sequence of SEQ ID NO: 1.

5. The collagenase according to claim 3, wherein said variants are **characterized by** amino acid substitutions, deletions or additions.

6. An isolated nucleic acid sequence, wherein said nucleic acid sequence encodes the collagenase according to any one of claims 1-5.

7. The isolated nucleic acid sequence according to claim 6, wherein said nucleic acid sequence is represented by SEQ ID NO:2.

8. A recombinant expression vector comprising an isolated nucleic acid sequence according to any one of claims 6-7.

9. A host cell transformed with the recombinant expression vector according to claim 8.

10. The host cell according to claim 9, wherein said host cell is of prokaryotic or eukaryotic origin.

11. The host cell according to claim 10, wherein said host cell of prokaryotic origin is derived from the *E. coli* Rosetta strain.

12. A method for the production of a collagenase, comprising cultivating the host cells of claim 9 to express the collagenase, and recovering the thus produced collagenase.

13. An isolated antibody that binds specifically to the collagenase according to claim 1.

14. Use of a collagenase according to any one of claims 1-5 for the isolation of cells from human or animal tissue.

15. Use of a collagenase according to any one of claims 1-5 for the preparation of a pharmaceutical composition for the treatment of diseases associated with excessive collagen deposition.

16. Use of a collagenase according to any one of claims 1-5 for the preparation of a pharmaceutical composition for the treatment of burns, ulcer, scab, white hard scab of collagen base, cheloid, necrosis and wounds.
